(19) Europäisches Patentamt
European Patent Office
Office européen des brevets

(11) EP 0 436 443 B1

## (12) EUROPEAN PATENT SPECIFICATION

(45) Date of publication and mention
of the grant of the patent:
**17.04.1996 Bulletin 1996/16**

(51) Int. Cl.$^6$: **C07C 205/06**, C07C 201/08,
C07C 205/12

(21) Application number: **90403813.0**

(22) Date of filing: **31.12.1990**

(54) **Nitration process**

Nitrierungsverfahren

Procédé de nitration

(84) Designated Contracting States:
**BE DE ES FR GB IT NL**

(30) Priority: **04.01.1990 US 461142**

(43) Date of publication of application:
**10.07.1991 Bulletin 1991/28**

(73) Proprietor: **NRM INTERNATIONAL
TECHNOLOGIES C.V.
NL-3512 AD Utrecht (NL)**

(72) Inventors:
• **Guenkel, Alfred A.
Burnaby, B.C. V5B 3M3 (CA)**

• **Rae, John M.
Vancouver, B.C. V6N 1Y8 (CA)**
• **Hauptmann, Edward G.
Vancouver, B.C. V7V 2N3 (CA)**

(74) Representative: **Kedinger, Jean-Paul et al
c/o Cabinet Malemont
42, avenue du Président Wilson
F-75116 Paris (FR)**

(56) References cited:
**EP-A- 0 039 556        EP-A- 0 373 966
US-A- 2 737 522        US-A- 3 780 116**

## Description

This invention relates to a continuous process to nitrate nitratable organic compounds, particularly aromatic hydrocarbons and halogenated aromatic hydrocarbons.

### DESCRIPTION OF THE PRIOR ART

Nitrated aromatic hydrocarbons and nitrated halogenated aromatic hydrocarbons are important chemical intermediates. Examples of this class of compounds produced by industry in large volumes are nitrobenzene (MNB), mononitrotoluene (MNT), dinitrotoluene (DNT), and mononitrochlorobenzene (MNC).

The technology of manufacturing these compounds is well established and is discussed in a number of books, for example in Kirk-Othmer, "Encyclopedia of Chemical Technology", 3rd Edition, 1978, Vol. 15 in the articles entitled "Nitration" and "Nitrobenzene and Nitrotoluenes".

Many industrial manufacturing processes for these compounds have been developed, yet nitration is still an active field of research as evidenced by the large number of patents regularly issued.

In the present specification discussion will be restricted largely to the production of MNB but this is a mere convenience. Advantages provided by the present invention can equally well be realized in the nitration of other aromatic hydrocarbons or halogenated aromatic hydrocarbons. It is well known that many of these nitrocompounds are manufactured on a campaign basis using common equipment and selecting for each compound to be nitrated specific feed rates, a suitable acid composition and an appropriate operating temperature.

It is generally believed that the reactive species in nitration is the positively charged nitronium ion, $NO_2$. The nitronium ion is present in strong nitric acid and in a certain concentration range of mixed acid, which is a common term in this art for an acid consisting of a blend of sulfuric and nitric acids. A ternary diagram of water, sulfuric acid and nitric acid showing the nitronium ion concentration in mixed acid is set out in the above volume 15 of Kirk-Othmer on page 843. The diagram reveals that, for example, MNB is nitrated essentially only in mixed acids of a composition where the nitronium ion is present, the limit of nitration of MNB coinciding approximately with the limit of spectroscopic detection of the nitronium ion. A second region of interest in this ternary diagram is the region of high sulfuric acid strengths where undissociated pseudo acid, $NO_2.OH$, is spectroscopically not detectable. In the ternary diagram this region can be approximately mapped by connecting three points A, B and C where A corresponds to a composition of about 82% sulfuric acid and 18% nitric acid, B corresponds to a composition of about 55% sulfuric acid and 45% water and C corresponds to 100% sulfuric acid.

In industrial nitration the formation of oxidation by-products is significant. For example in the nitration of benzene, the undesirable by-products dinitrophenol and picric acid are formed. The formation of by-products is, of course, undesirable in any industrial reaction. It is wasteful of the reactants and the by-products must also be separated from the desired product, frequently by the use of expensive reagents in expensive treatment facilities. The ideal situation is that the desired reaction proceed to the exclusion of side reactions, generally as rapidly as possible.

The exact mechanism of the nitration and oxidation reactions in aromatic, mixed acid nitrations and the kinetics of the reactions are difficult to assess accurately because the reactions take place in a heterogeneous system. The hydrocarbons and the nitrated hydrocarbons have generally a low solubility in the mixed acid and sulfuric acid and water are almost insoluble in aromatic hydrocarbons and nitrated aromatic hydrocarbons. Therefore, the reactions are thought to occur near the interface between the organic and acid phases. However, reactions in both the acid and hydrocarbon phase are possible and have been shown to occur. Nitric acid may diffuse into the organic phase since nitric acid is quite soluble in certain aromatic hydrocarbons even in the presence of sulfuric acid. Evidence of oxidation reactions in the organic phase during aromatic nitration is given in the literature, for example by Albright, et al., "Reactions Occurring in the Organic Phase During Aromatic Nitrations", J. Appl. Chem. Biotechnol., 26, 522, 1976.

Parameters affecting the rate of the reaction and the rate of formation of by-products in mixed acid nitration are the mixed acid strengths, temperature, molar ratio of nitric acid to hydrocarbon, agitation, and the species of hydrocarbon nitrated. In general, it is difficult in experimental analysis to separate the effect of the various parameters. Agitation is important in aromatic nitration not only to promote mass transfer to the interface between the acid and organic phases but also to create the interface by dispersing one of the phases into the other. Generally it is thought that the reaction rate increases only marginally with agitation, once a critical level of agitation has been achieved - Urbanski, "Chemistry and Technology of Explosives", Vol. 1, The MacMillan Company, New York, 1964, 153. Many laboratory nitrations are carried out under conditions of "vigorous stirring", postulating that mass transfer resistance has been limited. However, establishing whether a nitration reaction is kinetically controlled or a mass transfer controlled is usually not answered convincingly. In industry agitation is often dictated by the requirement of assuring adequate heat transfer and temperature control.

The effect of mixed acid composition on the nitration rate is two-fold. First the composition has an effect on the nitronium ion concentration, as previously discussed, and secondly it has an effect on the solubility of the hydrocarbons in the acid phase. As the nitration reaction is thought to take place near the interface, solubility of the hydrocarbon in the acid layer surrounding the hydrocarbon acid containing the reactive species, the nitronium ion, will have a significant effect on nitration rate at the interface. Data on hydrocarbon solubility are reported in the literature, for example in Urbanski, loc.cit. and at page 230. The relative rates of toluene and benzene nitration under heterogeneous conditions, as compared to the rates under homogeneous conditions, have been explained by reference to the difference in solubilities of toluene and benzene in mixed acid - Hanson et al. "Macrokinetics of Toluene and Benzene Nitration under Laminar Conditions", Chem. Eng. Sci, 32, 775, 1977. The molar ratio of nitric acid to hydrocarbon has a significant influence on rate and by-product formation, particularly in the case of continuous nitration processes. It is common knowledge that chemical reactions can be driven to completion within a reasonable time only if one of the reacting species is present in excess. In nitration one has the option of working with stoichiometic excess of nitric acid or with excess of nitratable aromatic hydrocarbon. When operating with excess nitric acid the spent acid, which is the common term in this art for a mixed acid essentially depleted of nitric acid, will contain unreacted nitric acid, which may have opportunity to diffuse into the organic phase giving rise to the oxidation side reactions. Also, the rate of formation of dinitrocompounds can be expected to be much higher as the mononitrated hydrocarbon will stay in contact with nitric acid and will dissolve nitric acid.

A model of aromatic nitration has been published in the literature by Albright, et al., "Industrial and Laboratory Nitrations", ACS Symposium Series 22, American Chemical Society, 1976, 201. The model defines the following steps in the nitration process:

i) non-nitrated hydrocarbon diffuses through the organic phase towards the interface
ii) from the interface the non-nitrated hydrocarbon diffuses into the bulk aqueous phase
iii) while diffusing into the aqueous phase the non-nitrated hydrocarbon reacts to form the nitrocompound
iv) the nitrocompound formed diffuses back through the aqueous phase to the interface
v) from the interface the nitrocompound diffuses into the bulk organic phase
vi) nitric acid diffuses from the bulk aqueous phase towards the interface, reacting with the non-nitrated hydrocarbon en route
vii) the water formed diffuses back into the bulk aqueous phase
viii) some nitric acid diffuses from the interface into the bulk organic phase

The model can accommodate all the previously discussed process parameters known to affect the nitration rate and the rate of by-product formation.

In conventional aromatic nitration the original batch nitrator process of benzene is described by Urbanski loc.cit. The nitrator is filled with benzene and the mixed acid is added gradually under vigorous agitation and temperature control. The process is operated with a slight excess of benzene and the product nitrobenzene will thus contain unreacted benzene. When operating with a slight excess of nitric acid, the spent acid and product MNB will contain nitric acid. By adding the mixed acid to the benzene, contact of a large quantity of nitric acid with nitrobenzene is avoided and the organic layer will be in contact mostly with spent acid. Continuous nitration of benzene is discussed by Groggins, "Unit Processes in Organic Synthesis", McGraw Hill International, 1958. Mixed acid or separate streams of nitric acid and strong sulfuric acid and benzene are fed to a nitrator provided with cooling coils and an agitator. The nitrator discharges to a second nitration stage or to a gravity settler. Mixed acid composition and spent acid composition are virtually the same as in the batch process. The continuous process can also operate with either excess benzene or excess nitric acid. Typical operating temperatures are in the range of 50 to 70°C, which is below the boiling point of benzene.

A major cost in the manufacture of nitrobenzene is the disposal or reconcentration of the spent acid. Reconcentration of the spent acid is an energy intensive process. Castner in U.S. Patent 2,256,999 proposed an adiabatic nitrobenzene process where the heat of nitration is used in the reconcentration of the spent sulfuric acid. However, a shortcoming of this process is pointed out in example 17 of U.S. Patent 4,021,498 to Alexanderson et al. The benzene fed into the adiabatic nitrator is in contact with a large excess of nitric acid at high temperatures for extended times resulting in the formation of excessive amounts of dinitrobenzene.

Continuous adiabatic nitrobenzene processes analogous to Castner's batch process are described and claimed in U.S. Patents 4,091,042 and 4,021,498, both to Alexanderson et al.. Both patents carefully specify operating temperatures and suitable mixed acid strengths. The nitrators are operated under pressure to avoid flashing of benzene. The main improvement of these patents lies in their successful integration of the continuous nitration process and the continuous vacuum sulfuric acid concentration process. The reconcentrated sulfuric acid required in the nitration can be produced at an economically feasible vacuum in the sulfuric acid concentrator. This process is further discussed by Guenkel et al. "Nitrobenzene via an Adiabatic Reaction", Chem. Eng., August 10, 1981.

An alternative approach to using the heat of nitration is discussed in U.S. Patents 3,928,475 to Dassel and 3,981,934 to McCall. In these processes a reaction vessel contains a mixed acid. Nitric acid is fed into the vessel continuously as is benzene vapour. Benzene and nitric acid react to form nitrobenzene. The water entering with the nitric acid and formed in the process is removed as a vapour stream together with benzene and nitrobenzene vapour.

These processes use the heat of nitration but some energy is wasted as a large volume of nitrobenzene and benzene is vaporized from the reaction vessel only to be condensed and recycled. A drawback of both processes appears to be the rate of formation of by-products, namely dinitrobenzene and dinitrophenol. Their rate of formation appears to be higher than in the process of Alexanderson et al..

A further approach to utilizing the heat of nitration for the reconcentration of the spent acid is disclosed in European Patent Application EP-A-39556 to McCall. In this process the nitration reaction and the evaporation of the water formed in the process and entering with the nitric acid again take place in a common vessel. Again, a drawback of the process appears to be the high rate of formation of dinitrophenol.

Attempts have been made to avoid the use of sulfuric acid altogether and to produce nitrobenzene by reacting benzene and nitric acid in the absence of sulfuric acid. This approach is discussed by Othmer et al., "Nitration of Benzene, Continuous Process Using Nitric Acid Alone", Ind. Eng. Chem., 34 No. 3, 286, 1942. Patents on a nitric acid only process include U. S. Patents 2,739,174 to Ross and 3,780,116 to Sahgal.

The main objection against a process using nitric acid alone is the fact that mixtures of nitric acid and benzene or nitrobenzene can detonate, making such a process potentially hazardous. Furthermore, data on by-product formation published in the literature show high levels of by-products - Kanhere, et al., "Nitration with Nitric Acid Alone: An Alternative to Mixed Acid Nitration", Indian Jour. Techn., 19, 319, Aug. 1981.

The concept of adiabatic nitration has been discussed in other patents but mainly in conjunction with recovery of small residual amounts of nitric acid left in the spent acid. The intention is to recover nitric acid by contacting the spent acid with the feed hydrocarbon. As residual levels of nitric acid are often very low, adiabatic operation is possible in the nitric acid extraction stage. Examples of such processes are U.S. Patents 2,773,911 to Dubois, et al.; 2,849,497 to Buchanan and 4,496,782 to Carr. A process for the adiabatic nitration of monochlorobenzene is disclosed in U.S. Patent 4,453,027 to Vaidyanathan.

In isothermal nitration processes agitation requirements are often dictated by the need for efficient heat transfer. This applies particularly to batch nitration processes where the nitrator may have a large volume. However, in an adiabatic process the heat of nitration is absorbed by the process fluid as sensible heat and transfer of heat to a cooling water loop is not required. Thus, in an adiabatic process agitation requirements are solely set by mass transfer requirements in the nitrator. Mixing in general and mixing in stirred tanks is discussed in a number os specialized books including Uhl, et al., "Mixing Theory and Practice", Vol 1, Academic Press. 1966; Nagata, "Mixing Principles and Applications", John Wiley & Sons, 1975; Oldshue, "Fluid Mixing Trechnology", McGraw-Hill, 1983. The disclosures of these publications are incorporated herein by reference.

Because in industrial nitrations the nitration reaction takes place at the interface between the hydrocarbon and acid phases, the diameter of the emulsion droplets in a nitrator is of great significance. For a given mass fraction of nitrated aromatic hydrocarbon the interfacial area changes as the reciprocal of the droplet diameter. While bulk mixing and the creation of an emulsion are important design considerations in nitration, small scale mixing or micromixing and the reactor type must also be evaluated. These subjects are covered by Levenspiel, Chemical Reaction Engineering, John Wiley & Sons, Inc., 1962. Distinction is made between a plug-flow reactor and a back-mix reactor, both being idealized reactor concepts. In the plug-flow reactor reactants entering at a given point in time stay in intimate contact and leave the reactor together. They have the same residence time and no mixing with material entering the reactor earlier or later will take place. A pipe reactor operating in the turbulent flow regime comes close in practice to realizing the ideal plug-flow reactor concept.

In the ideal back-mix reactor reactants entering the reactor at a given time are instantaneously dispersed producing a uniform mix in the reactor. The products leaving the back-mix reactor have the same composition as that of the process fluids in the bulk of the reactor. Reactants entering at a given time may be in contact with reactants entering earlier and later. A stirred tank reactor, a type of reactor commonly used in nitrations, is often thought to realize the concept of a back-mix reactor. This may be true in laboratory scale reactors, where agitation can be intense, and where turnover time of liquids in the reactor is short, but the concept is difficult to implement on the scale of commercial nitrators. Dispersion and mixing on a micro-scale in a commercial nitrator are relatively slow processes. Thus, it is to be expected that a mixed acid entering a commercial nitrator will not disperse instantaneously. Rather it will encounter partially emulsified hydrocarbon and nitrated hydrocarbon. Therefore, diffusion of nitric acid or pseudo acid into droplets of nitrocompounds may occur since nitrated hydrocarbon may not react with nitric acid or the nitronium ion which diffuses to the interface. As discussed above, nitric acid which is absorbed into the hydrocarbon or the nitrated hydrocarbon phase has the potential to give rise to the formation of oxidation by-products.

## SUMMARY OF THE INVENTION

The present invention provides a continuous process to nitrate a nitratable aromatic compound typically an aromatic hydrocarbon or a halogenated hydrocarbon, in which the formation of oxidation by-products is substantially reduced compared with the prior art.

Accordingly, the present invention provides a continuous process to nitrate a nitratable aromatic compound in a nitronium ion solution in a nitrator, the process comprising:

(a) introducing into the nitrator a nitronium ion solution of a composition that contains nitric acid, water and sulfuric acid, and lies within an area defined by three points A, B and C of a ternary phase diagram of nitric acid, sulfuric acid and water where:

A corresponds to 82% of sulfuric acid and 18% nitric acid;
B corresponds to 55% sulfuric acid and 45% water; and
C corresponds to 100% sulfuric acid;

said composition being selected so that nitric acid is no greater than 2.99% and is substantially fully dissociated to nitronium ion and the sulfuric acid strength is sufficiently high to permit a temperature at the inlet of the nitrator of from 110 to 120°C ;

(b) feeding the nitratable aromatic compound into the nitronium ion solution through an injection means able to create a fine emulsion of said aromatic compound within the nitronium ion solution with the aromatic compound being evenly distributed in the nitronium ion solution;

(c) causing the nitronium ion solution containing said nitratable aromatic compound to react in a plug-flow or pipe nitrator containing mixing elements able to achieve and to maintain mixing of the contents of the nitrator to nitrate the nitratable compound to provide an aromatic nitro compound containing only low levels of impurities.

The mixed acid composition of step (a) is characterized by the fact that pseudo acid, $NO_2.OH$, is spectroscopically not detectable. Thus, the mixed acid contains mostly nitronium ions, but possible also nitrate ions, $NO_3$, or various other ions combining nitric acid, sulfuric acid and water.

While the use of a plug-flow or a pipe nitrator and the dispersion of the hydrocarbon through an injection means or its equivalent is an important feature of the invention, there is some latitude in the location of the injection point of the various streams into the pipe nitrator. Depending on the hydrocarbon to be nitrated it may be either advantageous to pre-mix the acids of step (a) externally of the pipe nitrator or to inject the nitric acid and the sulfuric acid directly and separately into the nitrator. The optimum among the various choices of feeding the pipe nitrator depends on the type of hydrocarbon to be nitrated, on the mixed acid composition, and on the operating temperature and it must be determined experimentally. This can be done in the laboratory or by providing multiple connections to the full scale pipe nitrator.

The use of an injection means and a plug-flow type of nitrator of steps (b) and (c) is proposed as a means of avoiding back-mixing through which contact between nitrated or partially nitrated aromatic hydrocarbon and fresh mixed acid is avoided. Thus, diffusion of nitric acid or of pseudo acid into the organic phase is largely avoided.

BRIEF DESCRIPTION OF THE DRAWINGS

The invention is further illustrated in the drawings in which:

Figure 1 shows a droplet of dispersed hydrocarbon surrounded by a concentric shell of nitronium ion solution;
Figure 2 illustrates a first embodiment of the invention in which a nitration process is carried out in the isothermal mode;
Figure 3 illustrates a second embodiment of the invention in which a nitration process is carried out in the adiabatic mode;
Figure 4 illustrates an example of an apparatus used to carry out the nitration process;
Figure 5 is a section in the line 5-5 in Figure 4;
Figure 6 is a ternary phase diagram for the system water-nitric acid-sulfuric acid;
Figure 7 illustrates another example of an apparatus used to carry out the nitration process.
Figure 8 is a section in the line 8-8 in Figure 7; and
Figure 9 is a section of the ternary phase diagram of Figure 6.

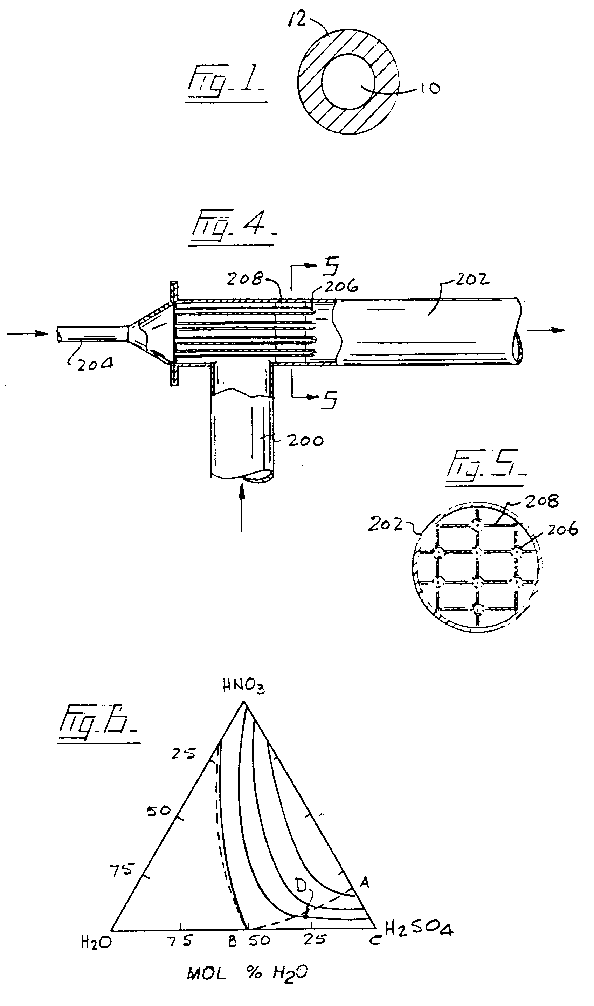

Referring now to Figure 1, which shows schematically a single hydrocarbon droplet 10 of diameter Di surrounded by a shell 12 of diameter Do of mixed acid, the nitration process on a microscale is visualized as follows:

i) Under operating conditions typical in an adiabatic nitration process the volumetric phase ratio of the hydrocarbon to nitric mixed acid phases could be about one to four. Thus, if each hydrocarbon droplet is thought to be surrounded by an "acid shell" the ratio of the diameters Di of the hydrocarbon to the diameter Do of the acid shell is 1/1.71. At a given volumetric fraction of the hydrocarbon this ratio doesn't depend on the size of the hydrocarbon droplet but it will vary if the volumetric fraction is changed. The lengths of the diffusion paths to the interface from the hydrocarbon phase and from the acid phase are of the same order of magnitude.

ii) Once a hydrocarbon droplet has been created, the nitration reaction will take place at the interface. This requires that the nitronium ion, $NO_2^+$ diffuses to the interface.

iii) When the nitration process is run with excess hydrocarbon on a molar basis, there will always be a large excess of unreacted hydrocarbon at the interface. At the start of the nitration the molar concentration of the unreacted hydrocarbon in the hydrocarbon phase is 100 percent while that of the nitronium ion in the acid phase could be about 5 percent or less. When the nitration reaction approaches completion the molar concentration of the nitronium ion approaches zero while there remains still an appreciable amount of unreacted hydrocarbon. Therefore, diffusion of hydrocarbon inside the hydrocarbon phase is not a significant factor in the overall reaction rate.

iv) If the hydrocarbon droplet is created in a mixed acid where nitric acid is present only as the reactive species, the nitronium ion, then diffusion of nitric acid or pseudo acid into the organic phase can't take place. Therefore, oxidation side reactions, other than those going through a mechanism involving the nitronium ion, are largely eliminated.

v) The nitration reaction at the interface is very fast so that neither the nitronium ion can penetrate into the hydrocarbon phase, nor can unreacted hydrocarbon penetrate more than a few molecular diameters into the acid phase except when the nitration reaction approaches completion or when the intrinsic homogeneous nitration rate is very slow. Any hydrocarbon initially present in the acid phase will be quickly nitrated, the acid phase will essentially be saturated with the nitrocompound during all stages of the nitration process.

vi) Dinitration, which is a much slower reaction than the mononitration will take place mostly in the acid phase as dissolved mononitro compounds react with the nitronium ion. Dinitration, a homogeneous, kinetically controlled process, competes for nitronium ions with mononitration, a process controlled by diffusion of the nitronium ion to the interface and by the solubility of the hydrocarbon in the acid phase. Dinitration can be controlled by altering the rate of mononitration which consumes the nitronium ion. This rate of mononitration can be altered by, for example, increasing the area of the interface by creating a finer emulsion using an appropriate spray injector.

vii) Bulk mixing is an important aspect of nitration in that it is responsible for the creation of small hydrocarbon droplets and in that it disperses the reactants fed into the nitrator. However, once a droplet has been created, the rate of nitration in its environment is dictated by diffusion in the acid shell surrounding each droplet. The droplet and the acid shell will, in general, stay together with occasional bulk mixing and coalescence of drops. Bulk mixing in a typical commercial scale nitrator of the back-mix type is a relatively slow process and, for this reason, there is ample opportunity for partially nitrated hydrocarbon to establish contact with the concentrated feed mixed acid. Such intimate contact may lead to diffusion of nitric acid or pseudo acid into the hydrocarbon phase, if those species are present and subsequent oxidation.

viii) Once the hydrocarbon has been dispersed uniformly in the acid phase, then the nitration reaction can be viewed as an unsteady process of mass transfer of the nitronium ion to the interface in the acid shell surrounding each hydrocarbon droplet.

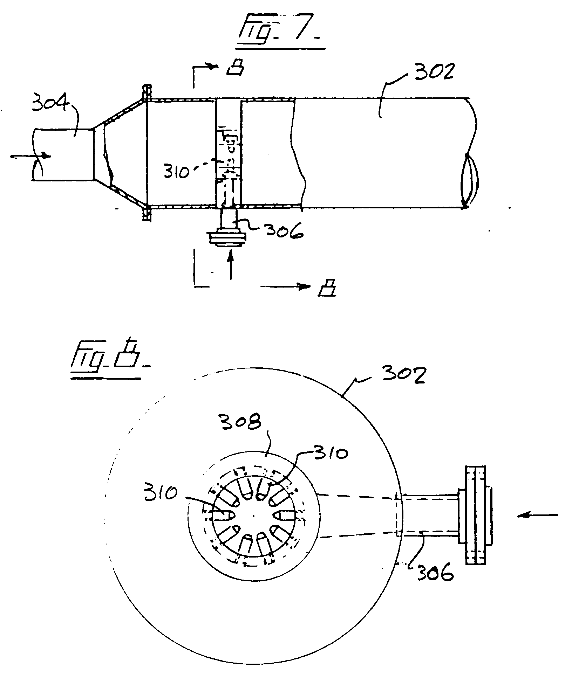

Figure 2 shows a flow diagram for an isothermal nitration process. A pipe nitrator 14 receives recycle spent sulfuric from conduit 16, reconcentrated sulfuric acid from conduit 18, nitric acid from conduit 20 and hydrocarbon to be nitrated from conduit 22. The acid streams are blended in a ratio such that the resulting mixed acid contains nitric acid mostly in the form of the reactive species, the nitronium ion and not as pseudo acid, $NO_2.OH$. Mixed acids where pseudo acid cannot be detached spectroscopically can approximately be mapped on the ternary diagram shown in Figure 6 by connecting the three points A, B and C. A corresponds to a composition of about 82% sulfuric acid and 18% nitric acid, B corresponds to a composition of about 55% sulfuric acid and 45% water and C corresponds to 100% sulfuric acid.

The solid lines in Figure 6 are lines of constant nitronium ion concentration, the dashed line in the right-hand corner maps the region where nitric acid is fully dissociated. Finally, a second dashed line running in close proximity to a solid line, the line suggesting zero nitronium ion concentration, delineates the limits of nitration of mononitrobenzene.

The three acid streams from conduits 16, 18 and 20 may be mixed in line before entering the pipe nitrator 14. Alternatively a slip stream of reconcentrated sulfuric acid can be introduced through conduit 24 and may be blended with a nitric acid supplied from conduit 26 and be injected separately from the recycle sulfuric acid.

The pipe nitrator 14 discharges to a stirred tank type of nitrator 28 having provision for cooling via conduit 30. The process fluids, nitrated hydrocarbon and spent acid flow from nitrator 28 to a gravity separator 32. The product nitrocompound is discharged from the separator 32 via conduit 34 while the spent acid is delivered via conduit 36 and is subsequently split into a recycle stream for conduit 16 and a stream for conduit 38 leading to a sulfuric acid concentrator 40. In the sulfuric acid concentrator 40 water is removed via conduit 45 while reconcentrated sulfuric acid is recycled through conduit 18. The sulfuric acid concentrator 40 may be omitted and in this case spent acid is disposed and strong sulfuric acid is imported.

It may be an advantage to install mixing 46 elements inside the pipe nitrator 14.

The discussion above in conjunction with Figure 1, where the mechanism of the nitration reaction is elucidated, shows the importance of creating a very fine spray, uniformly distributed over the cross section of the pipe nitrator.

Figure 3 shows a nitration process operating under adiabatic conditions. A pipe nitrator 100 receives reconcentrated sulfuric acid from conduit 102, nitric acid from conduit 104 and hydrocarbon to be nitrated from conduit 106. The acid streams are again blended in a ratio such that the resulting mixed acid contains nitric acid mostly in the form of the reactive species, the nitronium ion. The two acid streams from conduits 102 and 104 may be mixed in line before entering the pipe nitrator 100 or may be injected separately into the pipe nitrator 100. The pipe nitrator 100 discharges to a stirred tank type of nitrator 112. In cases where the reaction rate is very fast, which is specific to the hydrocarbon species to be nitrated and specific to the operating conditions, the reaction may go essentially to completion inside the pipe nitrator 100 so that the stirred tank type of nitrator 112 is not required. In cases where the nitration rate is slow the pipe nitrator 100 serves mainly as a hydrocarbon dispersion device. Fresh nitrating acid entering the stirred tank nitrator 112 is intimately mixed with fresh hydrocarbon. The process fluids pass to a separator 114 and product nitrocompound is discharged from separator 114 via conduit 116 while the spent acid is delivered to a sulfuric acid concentrator 118. In the sulfuric acid concentrator 118 water is removed via conduit 120 while reconcentrated sulfuric acid is recycled to the pipe nitrator 100 via conduit 102.

Again, it may be advantageous to install static mixing elements 122 inside the pipe nitrator 100.

A key difference between the isothermal process shown in Figure 2 and the adiabatic process of Figure 3 is that energy is rejected from the isothermal process through the cooling water of junction 30, while no energy is rejected from the adiabatic process. Therefore, the heat of nitration is available in the acid concentrator 118 of the adiabatic process.

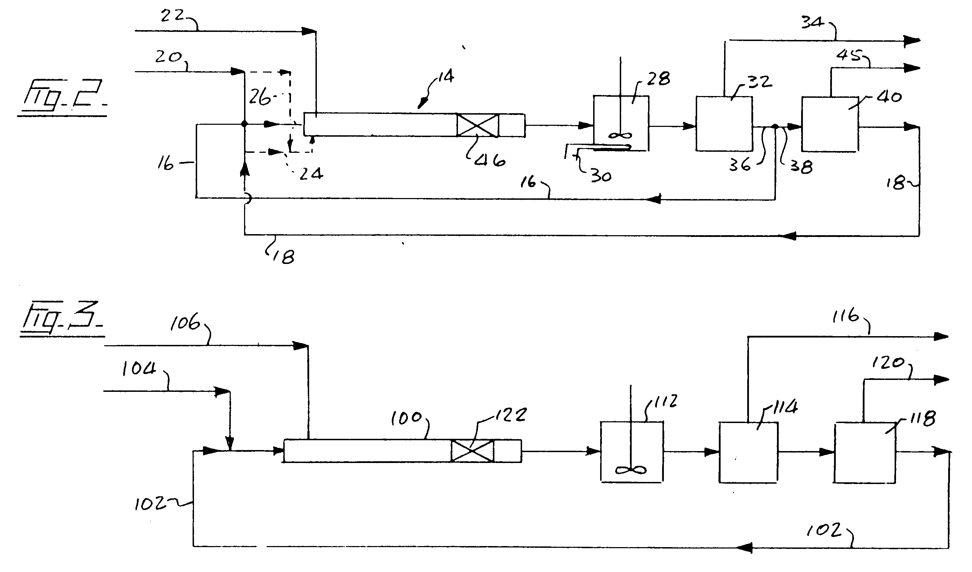

Figures 4 and 5 show an apparatus used to disperse the hydrocarbon into the acid phase. Acid is injected through a side-port 200 into a plug flow or pipe nitrator 202. The diameter of the pipe nitrator 202 is selected to establish flow in the turbulent regime which is usually assumed to prevail at Reynolds numbers larger than 4000. Into this turbulent acid stream inside the pipe nitrator 200 the hydrocarbon stream is injected through port 204 and is distributed through a number of evenly spaced spray injectors 206. The spray injectors 206 are small diameter pipes closed at one end but each provided with a number of spray nozzles which may be small holes drilled into the end. Baffles 208 are provided to stiffen the spray injectors 206 and to assure even distribution of the acid flow at the cross-section where the hydrocarbon enters the acid phase. Normally one spray injector 208 per 6.4516 cm$^2$ (square inch) of cross sectional area of the pipe nitrator 200 is sufficient. The number or distribution of the spray injectors 208 is dictated more by considerations relating to mechanical fabrication than by process requirements. The nitration process demands the creation of a hydrocarbon dispersion and an even distribution of the hydrocarbon within the constraints of practical fabrication techniques. The number and size of the spray nozzles provided are selected on the basis of common design practice, apparent to those skilled in the art.

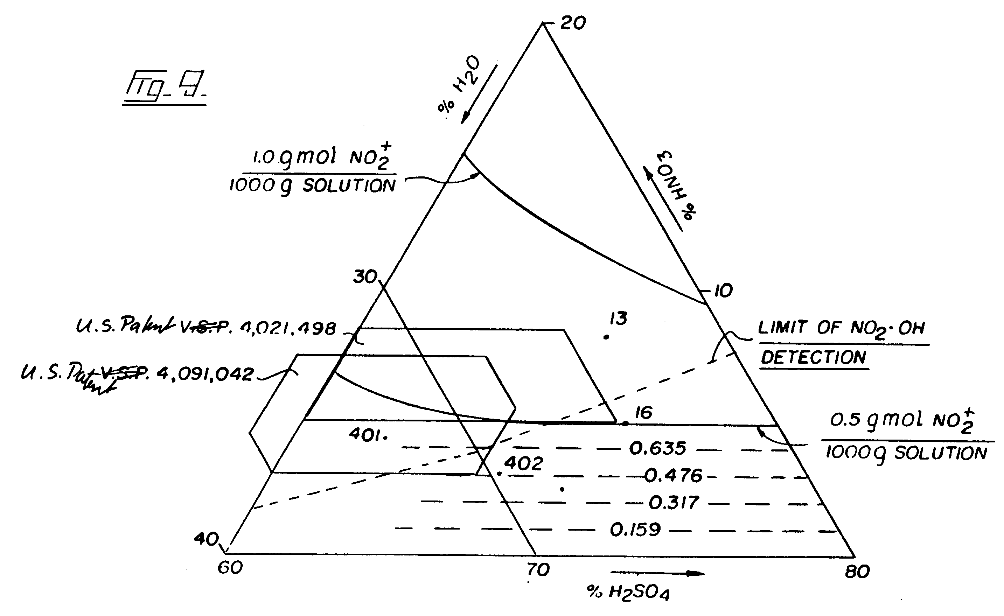

Other designs of the hydrocarbon injection systems are entirely feasible and it should be emphasized that Figure 4 essentially illustrates an example of a useful embodiment. Examples of further designs include a simple sparger ring or a sparger tube. Figures 7 and 8 show a plug flow nitrator 302 into which mixed acid flows through inlet 304. Benzene enters the reactor 302 through inlet 306 feeding to manifold 308 from which jets 310 pass benzene into the mixed acid. These jets 310 are small tubes into which holes are drilled in a pattern to achieve even distribution of benzene in the nitronium ion solution. Use of a plurality of jets 310 feeding benzene into a restricted portion of the nitrator ensures a rapid and efficient dispersion of the benzene in the mixed acid.

Figure 9 shows a section of the ternary phase diagram for the system nitric acid, sulfuric acid, water covering the range 60 - 80% $H_2SO_4$, 0-20% $HNO_3$ and 20-40% $H_2O$. Also shown are compositions of mixed acid claimed in the earlier patents of Alexanderson et al. Points 13 and 16 are said to fall outside the invention of U.S. Patent 4,021,498. Also shown in Figure 9 is a dashed line delineating the limits of spectroscopic detection of the pseudo acid $NO_2.OH$ and two curves giving the molar concentration of the nitronium ion solution determined spectroscopically. Finally, the horizontal dashed lines show the molar concentration of the nitronium ion if nitric acid were present only as nitronium ion. The concentrations are shown inserted into the dashed lines.

Points 401 and 402 of Figure 9 represents two mixed acids which were tested experimentally in a pipe nitrator of the present invention. Acid 401 represents a mixed acid of composition typically used in prior art processes while acid 402 falls outside the range claimed in prior art processes. Analysis of the crude MNB produced when operating with acid 401 showed the following level of by-product formation:

| 2,6 dinitrophenol | 60 ppm |
|---|---|
| 2,4 dinitrophenol | 1899 ppm |
| Picric acid | 1578 ppm |

Analysis of the crude MNB produced when operating with acid 302 showed the following level of by-product formation:

| 2,6 dinitrophenol | 281 ppm |
|---|---|
| 2,4 dinitrophenol | 2187 ppm |
| Picric acid | 797 ppm |

While there was small increase in the level of dinitrophenol, there was a drop by almost 50 percent of the level of picric acid when operating with acid 402. This result is surprising since prior art processes exclude operation in the range of acid 402.

While at first sight a shift in mixed acid composition from acid 401 to acid 402 seems minor, it is clearly significant in terms of the ion concentration. In the case of acid 401 only about 60% of the nitric acid is present in the form of nitronimum ion, whereas in the case of acid 402 no pseudo acid is present and about 75% of the nitric acid is present in the form of nitronimum ion. Thus, it seems of advantage to operate the adiabatic nitrobenzene process with mixed acids previously thought not suitable, in particular under conditions where pseudo acid, $NO_2.OH$ is spectroscopically not detectable and preferably under conditions where nitric acid is present only in the form of the nitronium ion.

Operating temperatures and acid strengths used in the process of this invention are set by consideration of kinetics and safety, both being specific to the hydrocarbon being nitrated. In an adiabatic nitration process the acid temperatures are also set by the requirements to match safe nitrating conditions with the operating temperatures of a sulfuric acid concentrator, typically a vacuum flash concentrator. Such a match has been disclosed in prior art processes, however, through the invention disclosed in this process it has become possible to operate at mixed acid strengths previously not considered feasible without producing excessive amounts of by-products. While it is entirely feasible to operate the process of this invention under conditions disclosed in prior art processes, it is preferred to operate at higher acid strengths than those claimed in prior art processes, thereby gaining the benefit of higher nitration rates and, surprisingly, lower rates of formation of by-products. In the specific case of the adiabatic nitrobenzene process it is feasible to reach the same conversion as that obtained in prior art processes requiring only a small fraction of the residence time required in the reactors of prior art processes. This result, which implies substantial capital savings, can be achieved while simultaneously reducing the rate of formation of by-products, in particular of picric acid.

Thus, the process of the present invention is characterized by a number of features not found in the prior art. The mixed acid composition in the present process is selected so that nitric acid is more fully dissociated to nitronium ion and the sulfuric acid strength is pushed as high as possible to take advantage of the higher nitration rates. A mixed acid useful in the process of the present invention would have the composition:

| | |
|---|---|
| $H_2SO_4$ | 72.02% |
| $HNO_3$ | 2.99% |
| $H_2O$ | 24.99% |

This mixed acid, basically a nitronium ion solution not containing any pseudo acid, serves merely as an example to demonstrate that the process of this invention can be operated with acids not previously known in the art. However, many other acid compositions may be used including acids proposed in prior art processes but preferably acids where the nitric acid is fully dissociated.

The strength of the reconcentrated sulfuric acid required to produce such a mixed acid would be about 75.6%. Figure 6 shows the region for complete nitric acid dissociation which is obtained for compositions falling at the lower right hand corner in the area of the diagram defined by points A, B and C falling below the dashed line. Point D is the composition of an example of a nitronium ion solution suitable to be used in the process of the present invention.

The residence time in the process of the present invention can be of the order of 25 seconds or less. This residence time is much shorter than that used in other processes. The short residence time is possible in the invention because:

i) the nitrator inlet temperature can be higher than in the earlier processes, about 110 to 120°C compared with about 95°C in the prior art.

ii) the strength of the reconcentrated sulfuric acid can be much higher than that used in earlier processes, about 75% sulfuric acid compared with about 68% in the prior art.

iii) the benzene is injected through spray nozzles which are uniformly distributed over the cross section of the plug-flow nitrator and are designed to create very small droplets.

iv) further dispersion of the hydrocarbon can be achieved through the use of mixing elements in a plug-flow reactor. In a particularly preferred embodiment that plug-flow reactor is as described and claimed in United States Patent Application Serial No. 405,930 filed September 12, 1989, the subject matter of which is incorporated herein by reference.

In the process of the present invention the nitrator operates under the static and dynamic head of the nitrobenzene acid separator which operates at atmospheric pressure but in the prior art the nitrator is pressurized to avoid flashing of benzene. The dynamic head loss through this plug-flow reactor can be established to avoid flashing of benzene. When the organic phase, mostly nitrobenzene, reaches the separator, its vapour pressure will be well below atmospheric. This is achieved by control of the final nitrator temperature through the amount of sulfuric acid circulating through the system.

Nitrogen oxides formed in the nitrators through oxidation side reactions are vented from the nitrobenzene-acid separator of this invention which may operate at atmospheric pressure, thus reducing the inerts load of the vacuum sulfuric acid concentrator. In the prior art these nitrogen oxides stay dissolved in the spent sulfuric acid going to the sulfuric acid concentrator where they contribute to the load on the vacuum system.

In the process of the present invention the energy required to maintain sulfuric acid strength in the nitration loop is supplied by preheating the nitric acid and benzene feeds to the nitrator using waste heat available in the washing and stripping areas of the plant. A start-up heater required in the nitration loop to bring the temperature of the circulating sulfuric acid up to its normal operating temperature can be very small. Its design basis is not set by the energy requirements of the nitration and acid concentration loop, but is rather dictated by the time necessary to bring the plant up to operating temperature.

The formation of dinitrobenzene in the process of the present invention can be controlled by adjusting the amount of excess benzene fed to the process. In the competing reaction of benzene and nitrobenzene, all nitric acid is consumed by the benzene before appreciable amounts of dinitrobenzene can form.

The rate of formation of dinitrophenol and picric acid can be controlled by limiting the maximum temperature in the nitration loop. This maximum temperature will be in the range of 135 to 145°C. This maximum temperature is selected to keep the vapour pressure of the process fluids in the nitrobenzene-acid separator below 1.01325 x $10^5$ Pa (760 mm of mercury) so that this separator can be operated at atmospheric pressure. Thus, the maximum operating temperature will depend on the spent acid composition and its vapour pressure and on the amount of excess benzene used and the partial pressure exerted by the benzene and nitrobenzene.

The rate of formation of and picric acid can also be reduced by elimination of contact between nitric acid and nitrated aromatic. As mentioned, the mixed acid composition is selected to ensure complete dissociation of the nitric acid, the mixed acid is in fact a nitronium ion solution. Thus, no pseudo acid will be able to diffuse into the aromatic phase, where the oxidation reactions are known to occur. In prior art processes nitric acid is only partially dissociated.

Both the rate of dinitrobenzene and of oxidation by-products formation are reduced by the use of a plug-flow nitrator and by even distribution and fine dispersion of the benzene over the cross-section of the nitrator. Thus, contact of nitrated material with fresh nitronium ion solution or undissociated nitric acid is largely avoided.

## Claims

1. A continuous process to nitrate a nitratable aromatic compound in a nitronium ion solution in a nitrator, the process comprising:

(a) introducing into the nitrator a nitronium ion solution of a composition that contains nitric acid, water and sulfuric acid, and lies within an area defined by three points A, B and C of a ternary phase diagram of nitric acid, sulfuric acid and water where:

A corresponds to 82% of sulfuric acid and 18% nitric acid;
B corresponds to 55% sulfuric acid and 45% water; and
C corresponds to 100% sulfuric acid;

said composition being selected so that nitric acid is no greater than 2.99% and is substantially fully dissociated to nitronium ion and the sulfuric acid strength is sufficiently high to permit a temperature at the inlet of the nitrator of from 110 to 120°C;
(b) feeding the nitratable aromatic compound into the nitronium ion solution through an injection means able to create a fine emulsion of said aromatic compound within the nitronium ion solution with the aromatic compound being evenly distributed in the nitronium ion solution;
(c) causing the nitronium ion solution containing said nitratable aromatic compound to react in a plug-flow or pipe nitrator containing mixing elements able to achieve and to maintain mixing of the contents of the nitrator to nitrate the nitratable compound to provide an aromatic nitro compound containing only low levels of impurities.

2. A process as claimed in claim 1 in which the nitronium ion solution is formed by mixing sulfuric and nitric acids prior to introduction into the nitrator.

3. A process as claimed in claim 1 where the nitric acid and the sulfuric acid are injected into the nitrator separately.

4. A process as claimed in claim 1 where part of the spent sulfuric acid discharged from the nitrator is separated from the hydrocarbon phase, recycled to the nitrator and the remainder is reconcentrated or disposed of and replaced by strong make-up sulfuric acid.

5. A process as claimed in claim 1 in which fluids discharged from the nitrator are sent to a back-mix nitrator to complete the nitration.

6. A process as claimed in claim 1 where fluids discharged from the nitrator are sent to a gravity settler for separation into an acid layer and a hydrocarbon layer.

7. A process as claimed in claim 1 where the heat of nitration and the heat of mixing are removed from the nitrator by external cooling.

8. A process as claimed in claim 1 in which part of the sulfuric acid is recycled sulfuric acid.

9. A process as claimed in claim 1 in which the spent sulfuric acid from the process is reconcentrated by flashing in a vacuum and is recycled to the nitration process.

10. A process as claimed in claim 1 in which the nitratable compound is fed into the process in molar excess of between 0.5 and 25 percent relative to the nitric acid.

11. A process as claimed in claim 1 in which the nitratable aromatic compound is selected from the group consisting of benzene, toluene, dimethylbenzene, and halogen derivatives, and mononitro derivatives.

12. A process as claimed in claim 1 in which the nitratable compound and feed nitric acid are preheated prior to feeding to the nitrator.

13. A process as claimed in claim 12 in which the nitratable compound is partially vaporized prior to feeding to the nitrator.

14. A process as claimed in claim 1 in which the nitratable compound is preheated prior to feeding it to the nitrator.

15. A process as claimed in claim 1 in which the nitric acid is pre-heated prior to feeding it to the nitrator.

16. The process of claim 1 wherein said composition is 72.02% sulfuric acid, 2.99% nitric acid, and 24.99% water.

**Patentansprüche**

1. Kontinuierliches Verfahren zum Nitrieren einer nitrierbaren aromatischen Verbindung in einer Nitroniumionenlösung in einem Nitrierapparat, wobei das Verfahren umfaßt:

    (a) Einbringen einer Nitroniumionenlösung von einer Zusammensetzung, die Salpetersäure, Wasser und Schwefelsäure enthält und innerhalb eines Bereichs liegt, der durch drei Punkte A, B und C eines ternären Phasendiagramms von Salpetersäure, Schwefelsäure und Wasser definiert ist, wobei:

        A 82% Schwefelsäure und 18% Salpetersäure entspricht;
        B 55% Schwefelsäure und 45% Wasser entspricht; und
        C 100% Schwefelsäure entspricht;

    wobei die Zusammensetzung so ausgewählt ist, daß die Salpetersäure nicht mehr als 2,99% beträgt und im wesentlichen vollständig in Nitroniumionen dissoziiert ist und die Schwefelsäurekonzentration ausreichend hoch ist, um eine Temperatur am Einlaß des Nitrierapparates von 110 bis 120°C zu gestatten,
in den Nitrierapparat;

    (b) Eintragen der nitrierbaren aromatischen Verbindung in die Nitroniumionenlösung durch ein Injektionsmittel, welches eine feine Emulsion der aromatischen Verbindung in der Nitroniumionenlösung erzeugen kann, wobei die aromatische Verbindung gleichmäßig in der Nitroniumionenlösung verteilt ist;
    (c) Bewirken, daß die Nitroniumionenlösung, welche die nitrierbare aromatische Verbindung enthält, in einem Kolbenstrom- oder Rohrnitrierapparat reagiert, welcher Mischelemente enthält, welche das Vermischen der Inhalte des Nitrierapparates herbeiführen und aufrechterhalten können, um die nitrierbare Verbindung zu nitrieren, um eine aromatische Nitroverbindung zu ergeben, welche nur niedrige Gehalte an Verunreinigungen enthält.

2. Verfahren nach Anspruch 1, in welchem die Nitroniumionenlösung durch Vermischen von Schwefelsäure und Salpetersäure vor dem Einbringen in den Nitrierapparat gebildet wird.

3. Verfahren nach Anspruch 1, worin die Salpetersäure und die Schwefelsäure getrennt in den Nitrierapparat injiziert werden.

4. Verfahren nach Anspruch 1, worin ein Teil der verbrauchten Schwefelsäure, die aus dem Nitrierapparat ausgetragen wird, von der Kohlenwasserstoffphase abgetrennt und in den Nitrierapparat zurückgeführt wird und der Rest aufkonzentriert oder entsorgt und durch konzentrierte Nachfüll-Schwefelsäure ersetzt wird.

5. Verfahren nach Anspruch 1, in welchem aus dem Nitrierapparat ausgetragene Flüssigkeiten in einen Rückvermischungs-Nitrierapparat überführt werden, um die Nitrierung zu vervollständigen.

6. Verfahren nach Anspruch 1, worin aus dem Nitrierapparat ausgetragene Flüssigkeiten in einen Schwerkraftabscheider zur Trennung in eine Säureschicht und eine Kohlenwasserstoffschicht überführt werden.

7. Verfahren nach Anspruch 1, worin die Nitrierungswärme und die Mischungswärme von dem Nitrierapparat durch äußere Kühlung abgeführt werden.

8. Verfahren nach Anspruch 1, in welchem ein Teil der Schwefelsäure recyclierte Schwefelsäure ist.

9. Verfahren nach Anspruch 1, in welchem die verbrauchte Schwefelsäure aus dem Verfahren durch Schnellverdampfen in einem Vakuum aufkonzentriert und in das Nitrierverfahren zurückgeführt wird.

10. Verfahren nach Anspruch 1, in welchem die nitrierbare Verbindung in einem molaren Überschuß zwischen 0,5 und 25 Prozent, bezogen auf die Salpetersäure, in das Verfahren eingetragen wird.

11. Verfahren nach Anspruch 1, in welchem die nitrierbare aromatische Verbindung gewählt ist auf der Gruppe bestehend aus Benzol, Toluol, Dimethylbenzol und Halogenderivaten und Mononitroderivaten.

12. Verfahren nach Anspruch 1, in welchem die nitrierbare Verbindung und die Salpetersäurebeschickung vor dem Eintragen in den Nitrierapparat vorgeheizt werden.

13. Verfahren nach Anspruch 12, in welchem die nitrierbare Verbindung vor dem Eintragen in den Nitrierapparat teilweise verdampft wird.

14. Verfahren nach Anspruch 1, in welchem die nitrierbare Verbindung vor dem Eintragen in den Nitrierapparat vorgeheizt wird.

15. Verfahren nach Anspruch 1, in welchem die Salpetersäure vor dem Eintragen in den Nitrierapparat vorgeheizt wird.

16. Verfahren nach Anspruch 1, worin die Zusammensetzung 72,02% Schwefelsäure, 2,99% Salpetersäure und 24,99% Wasser enthält.

**Revendications**

1. Procédé continu de nitration d'un composé aromatique apte à être nitré dans une solution contenant des ions nitronium à l'intérieur d'un appareil de nitration, le procédé comprenant les étapes qui consistent à :

(a) introduire dans l'appareil de nitration une solution contenant des ions nitronium, d'une composition contenant de l'acide nitrique, de l'eau et de l'acide sulfurique, et se situant dans une zone définie par trois points A, B et C d'un diagramme de phases ternaire d'acide nitrique, d'acide sulfurique et d'eau dans lequel :

A correspond à 82% d'acide sulfurique et 18% d'acide nitrique;
B correspond à 55% d'acide sulfurique et 45% d'eau; et
C correspond à 100% d'acide sulfurique;

ladite composition étant sélectionnée pour que l'acide nitrique ne représente pas plus de 2,99% et soit sensiblement complètement dissocié en ion nitronium et pour que la concentration d'acide sulfurique soit suffisamment élevée pour permettre une température de 110 à 120°C à l'entrée de l'appareil de nitration;

(b) injecter le composé aromatique apte à être nitré dans la solution contenant des ions nitronium par un moyen d'injection apte à créer une émulsion fine dudit composé aromatique au sein de la solution contenant des ions nitronium, le composé aromatique étant réparti de manière uniforme dans la solution contenant des ions nitronium;

(c) amener la solution contenant des ions nitronium et contenant ledit composé aromatique apte à être nitré à réagir dans un appareil de nitration à écoulement idéal ou tubulaire contenant des éléments de mélange capables de réaliser un mélange et de maintenir à l'état de mélange le contenu de l'appareil de nitration pour nitrer le composé apte à être nitré afin de former un composé nitré aromatique ne contenant que de faibles quantités d'impuretés.

2. Procédé tel que défini dans la revendication 1, selon lequel la solution contenant des ions nitronium est formée par mélange d'acides sulfurique et nitrique préalablement à son introduction dans l'appareil de nitration.

3. Procédé tel que défini dans la revendication 1, selon lequel l'acide nitrique et l'acide sulfurique sont injectés séparément dans l'appareil de nitration.

4. Procédé tel que défini dans la revendication 1, selon lequel une partie de l'acide sulfurique usé, évacué de l'appareil de nitration est séparée de la phase hydrocarbure et recyclée dans l'appareil de nitration, tandis que le reste est reconcentré ou jeté et remplacé par de l'acide sulfurique concentré d'appoint.

5. Procédé tel que défini dans la revendication 1, selon lequel des fluides évacués de l'appareil de nitration sont envoyés dans un appareil de nitration à mélange à contrecourant pour achever la nitration.

6. Procédé tel que défini dans la revendication 1, selon lequel des fluides évacués de l'appareil de nitration sont envoyés dans un décanteur à gravité pour être séparés en une couche d'acide et une couche d'hydrocarbure.

7. Procédé tel que défini dans la revendication 1, selon lequel la chaleur de nitration et la chaleur de mélange sont éliminées de l'appareil de nitration par un refroidissement extérieur.

8. Procédé tel que défini dans la revendication 1, selon lequel une partie de l'acide sulfurique est de l'acide sulfurique recyclé.

9. Procédé tel que défini dans la revendication 1, selon lequel l'acide sulfurique usé résultant du procédé est reconcentré par détente sous vide et recyclé dans le procédé de nitration.

10. Procédé tel que défini dans la revendication 1, selon lequel le composé apte à être nitré est mis en oeuvre dans le procédé avec un excédent molaire qui se situe entre 0,5 et 25 pour cent par rapport à l'acide nitrique.

11. Procédé tel que défini dans la revendication 1, selon lequel le composé aromatique apte à être nitré est choisi dans le groupe constitué par le benzène, le toluène, le diméthylbenzène, et les dérivés halogénés, et les dérivés mononitrés.

12. Procédé tel que défini dans la revendication 1, selon lequel le composé apte à être nitré et l'acide nitrique d'apport sont préchauffés avant d'être introduits dans l'appareil de nitration.

13. Procédé tel que défini dans la revendication 12, selon lequel le composé apte à être nitré est vaporisé partiellement avant d'être introduit dans l'appareil de nitration.

14. Procédé tel que défini dans la revendication 1, selon lequel le composé apte à être nitré est préchauffé avant d'être introduit dans l'appareil de nitration.

15. Procédé tel que défini dans la revendication 1, selon lequel l'acide nitrique est préchauffé avant d'être introduit dans l'appareil de nitration.

16. Procédé tel que défini dans la revendication 1, selon lequel ladite composition contient 72,02% d'acide sulfurique, 2,99% d'acide nitrique, et 24,99% d'eau.

_Fig. 1._

_Fig. 4._

_Fig. 5._

_Fig. 6._

HNO₃

MOL % H₂O

Fig. 2

Fig. 3

_Fig. 7._

304
302
310
306
B
B

_Fig. 8._

302
308
310
310
306

Fig. 4.